Europäisches Patentamt

European Patent Office

Office européen des brevets

(11) Publication number: 0 447 710 A1

# EUROPEAN PATENT APPLICATION

(21) Application number: 90310850.4

(22) Date of filing: 04.10.90

(51) Int. Cl.⁵: A61B 5/00

(30) Priority: 19.03.90 JP 69041/90

(43) Date of publication of application:
25.09.91 Bulletin 91/39

(84) Designated Contracting States:
AT CH DE ES FR GB IT LI SE

(71) Applicant: Makitsubo, Takeshi
14-6, 1-chome, Hikari-machi, Higashi-ku
Hiroshima-shi, Hiroshima-ken(JP)

(72) Inventor: Makitsubo, Takeshi
14-6, 1-chome, Hikari-machi, Higashi-ku
Hiroshima-shi, Hiroshima-ken(JP)

(74) Representative: Senior, Alan Murray et al
J.A. KEMP & CO 14 South Square Gray's Inn
London WC1R 5EU(GB)

(54) Electrocardiographic data transmission system.

(57) An electrocardiographic data transmission system using a telephone equipment includes an electrocardiograph (6) detecting a body surface potential signal varying with heart beats of the subject person (1) and outputting a sound pressure signal converted from the body surface potential signal into the telephone equipment (3), and a signal converter (10) coupled to the telephone equipment (5) of the specified hospital (4) receiving the sound pressure signal from the electrocardiograph (6), and outputting the body surface potential signal converted from the sound pressure signal to the display (11, 12). The system enables immediate diagnosis by transmitting the electrocardiographic data of the subject person (1) who falls into an urgent condition outside of the hospital (4) via telephone equipment (3, 5, 7, 8, 9) in real time to the cardiographic data analyzer (11) of the hospital.

The invention relates to an electrocardiographic data transmission system, in particular using telephone equipment to transmit the body surface potential of the subject person outside of a hospital via telephone line to an electrocardiogram analyzer of a hospital in real time which displays the electrocardiographic wave, thus enabling a doctor of the hospital to diagnose the circulatory function of the subject person.

Conventionally, a Holter cardiograph is used when the circulatory function of the subject person is diagnosed with an electrocardiogram. With the Holter cardiograph, it is necessary to record on the electromagnetic tape of the Holter cardiograph the cardiographic data of the subject person for 24 hours or 48 hours in succession, set the tape on the electrocardiogram regenerator installed in the hospital to regenerate the cardiographic data and display the cardiographic wave.

The recording means of the cardiographic data, as described in the above, needs a long time for recording and regeneration of the tape. Therefore, the problem is that it cannot cope with an urgent situation since a long lapse occurs before starting diagnosis of circulatory function of the subject matter.

According to the present invention there is provided an electrocardiographic data transmission system for use with telephone equipment comprising:

an electrocardiograph provided with electrodes for detecting a body surface potential signal varying with heart beats of a subject person and outputting a sound pressure signal converted from the detected body surface potential signal and which can be transmitted via a telephone;

a signal converter for connection to telephone equipment to receive the sound pressure signal transmitted via the telephone system from the electrocardiograph and outputted from the receiving telephone equipment and to output a body surface potential signal converted from the sound pressure signal; and

a display which receives the body surface potential signal outputted from the signal converter and displays the electrocardiographic wave of the subject person.

Thus the invention provides means enabling immediate diagnosis by transmitting the electrocardiographic data of the subject person via telephone equipment in real time to the cardiographic data analyzer of the hospital.

The system may also include a first telephone equipment at the side of the subject person for transmitting via a telephone line the sound pressure signal from the electrocardiograph set on a telephone transmitter, and a second telephone equipment for receiving the sound pressure signal in correspondence with the call from the telephone equipment at the side of the subject person via the telephone line.

Conveniently the cardiograph is adapted to be attachable to a telephone transmitter with one touch e.g. by press-fitting over the mouthpiece.

According to the electrocardiographic wave signal transmission system using the telephone equipment constituted as above, the family of the subject person who complains suddenly a pain in the chest, for example, calls up the telephone of the specified hospital for diagnosis of the subject person via the subject person's home telephone, telling to transmit the body surface potential signal of the subject person. Then, the electrocardiograph is set on the transmitter of the subject person's home telephone with one touch and the electrocardiograph is held to the chest of the subject person. The electrocardiograph detects the body surface potential signal varying in accordance with the heart beats, converts the signal to the sound pressure signal and inputs to the transmitter. This sound signal is transmitted to the telephone equipment of the hospital in the form of an electric signal transmittable via telephone line. The receiver of the electric signal in the hospital is set on a signal converter and the receiver outputs the sound pressure signal to the signal converter. The signal converter being inputted the sound pressure signal converts the sound pressure signal to the body surface potential signal, outputting to the display which displays the electrocardiographic wave of the subject person, thus enabling to analyze the circulatory function and diagnose the subject person.

DESCRIPTION OF THE PREFERRED EMBODIMENT

An embodiment of the invention is now explained with reference to a system diagram which illustrates an overall constitution of an electrocardiographic data transmission system using a telephone equipment.

When a subject person 1 happens to complain a pain in the chest, for example, the system of the invention functions as follows;

Using a telephone equipment 3 in a home 2 of the subject person 1, a telephone equipment 5 of a hospital 4 diagnosing the subject person 1 is called up, telling that the body surface potential signal of the subject person 1 is immediately transmitted as the electrocardiographic data. Then, an electrocardiograph 6 is set on a telephone transmitter 3A of the telephone 3, holds the electrocardiograph 6 to the chest of the subject person 1, detects the body surface potential as an electrocardiographic data, converts the signal to the sound pressure signal which can be transmitted via the telephone equipment 3, inputs the sound pressure signal to the telephone transmitter 3A, thus transmitting to the telephone equipment 5 of the hospital 4 via the transmitter 3A, the telephone 3, telephone line 7, telephone exchange 8, and telephone line 9. The sound pressure signal is outputted from the receiver 5A of the telephone 5, after answering the call from the telephone 3, and inputted to a signal converter 10 connected with the receiver 5A of the telephone 5. The sound pressure signal is demodulated to the body surface potential of the subject person 1 via the signal converter 10. The demodulated body surface potential of the subject person 1 is inputted to an electrocardiogram analyzer 11 to display the electrocardiogram of the subject person 1 and analyze the status of circulatory function.

In the system functioning as above, the electrocardiograph 6 is provided with electrodes to detect the body surface potential signal varying in accordance with heart beats of the subject person 1, converting the body surface potential signal detected to the sound pressure signal transmittable via the telephone equipment 3 and outputting the sound pressure signal. The cardiograph can be set on the transmitter 3A of the telephone equipment 3 with one touch and electric cells are contained as power source therein.

The signal converter 10 is constituted to be easily connected with the receiver 5A of the telephone equipment 5 and to convert the sound pressure signal outputted from the receiver 5A to the electric signal using, for example, piezoelectric elements of high precision.

The electrocardiogram 11 is connected with a printer 12 so that the cardiogram according to the body surface potential signal of subject person 1 may be printed out. In accordance with the analysis by the electrocardiogram analyzer 11 or examining the cardiogram printed out, the circulatory function for the subject person 1 can be diagnosed.

According to the invention described in the above, the body surface potential signal of the subject person can be transmitted to the electrocardiographic data analyzer of the hospital via the telephone equipment, therefore a doctor of the hospital can diagnose the subject person who falls into an urgent condition outside of the hospital.

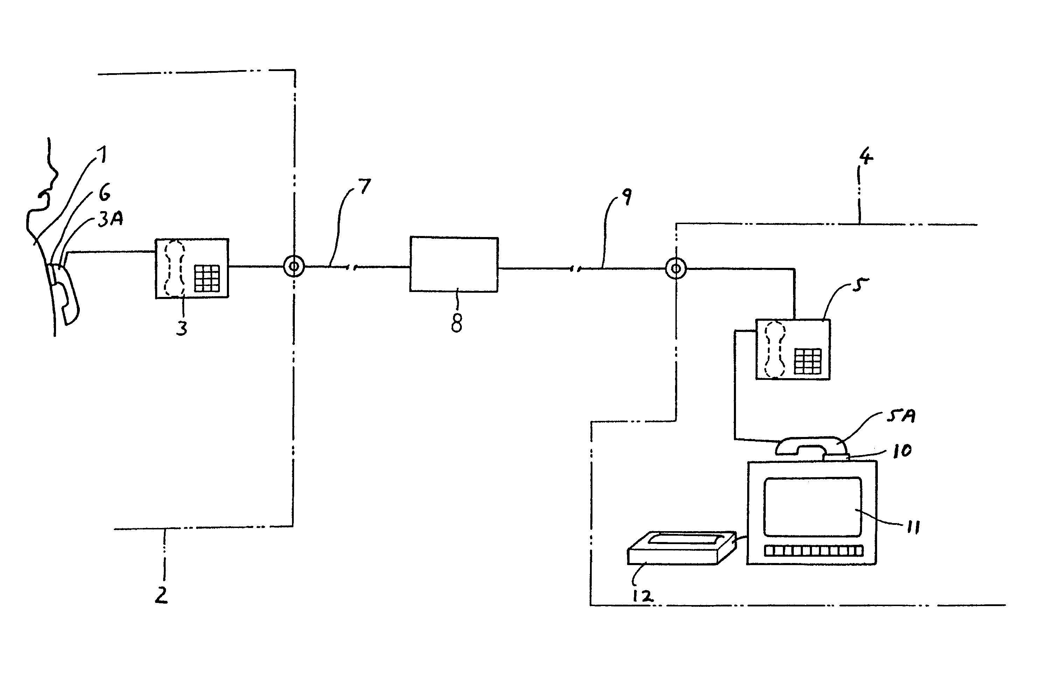

**Claims**

1. An electrocardiographic data transmission system for use with telephone equipment comprising:

    an electrocardiograph (6) provided with electrodes for detecting a body surface potential signal varying with heart beats of a subject person (1) and outputting a sound pressure signal converted from the detected body surface potential signal and which can be transmitted via a telephone (3);

    a signal converter (10) for connection to telephone equipment (5) to receive the sound pressure signal transmitted via the telephone system (7, 8, 9) from the electrocardiograph (6) and outputted from the receiving telephone equipment (5) and to output a body surface potential signal converted from the sound pressure signal; and

    a display (11, 12) which receives the body surface potential signal outputted from the signal converter (10) and displays the electrocardiographic wave of the subject person (1).

2. An electrocardiographic data transmission system according to claim 1, further comprising a first telephone equipment (3) at the side of the subject person for transmitting via a telephone line (7, 9) the sound pressure signal from the electrocardiograph (6) set on a telephone transmitter (3A), and a second telephone equipment (5) for receiving the sound pressure signal in correspondence with the call from the telephone equipment (3) at the side of the subject person (1) via the telephone line.

3. An electrocardiographic data transmission system according to claim 1 or 2 wherein the cardiograph (6) is adapted to be attachable to a telephone transmitter (3A) with one touch.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | BELL LABORATORICS RECORD, vol. 44, no. 2, February 1966, pages 42-47; J.L. CROUCH et al.: "Electrocardiograms by telephone" * Page 43, line 1 - page 45, line 35; page 46, line 34 - page 47, line 12 * | 1-3 | A 61 B 5/00 |
| X | EP-A-0 122 888 (GE.SV.IN. Srl) * Abstract; page 1, line 20 - page 3, line 9, page 4, lines 3-12; figure 1 * | 1-3 | |
| X | US-A-4 097 691 (S.J. EHRLICH et al.) * The whole document * | 1-3 | |

|  |
|---|
| TECHNICAL FIELDS SEARCHED (Int. Cl.5) |
| A 61 B |

The present search report has been drawn up for all claims

| Place of search | Date of completion of search | Examiner |
|---|---|---|
| The Hague | 14 June 91 | FONTENAY P.H.E.V. |